# EUROPEAN PATENT APPLICATION

(11) **EP 4 781 918 A1**
(43) Date of publication of application: **29.07.2026**
(21) Application number: 26154023.1
(22) Date of filing: 26.01.2026
(51) Int. Cl.: A61B 8/08, A61B 8/12, A61B 8/00, A61B 6/50

(54) **SYSTEMS AND METHODS FOR TRACKING AND DISPLAYING TISSUE THICKNESS**

(30) Priority: 27.01.2025 US 202519038639
(71) Applicant: Biosense Webster (Israel) Ltd., 2066717 Yokneam (IL)
(72) Inventor: BREM, Erez, 2066717 Yokneam (IL); ZIV-ARI, Morris, Irvine, 92618 (US); URMAN, Roy, 2066717 Yokneam (IL); HAIMAN, Guy, 2066717 Yokneam (IL); BARNEA, Nadav, 2066717 Yokneam (IL)
(74) Representative: Carpmaels & Ransford LLP

(57) **Abstract**

Methods may include receiving, at a computing device, at least one coordinate set associated with a catheter tip. Methods may include receiving, at the computing device, at least one three-dimensional (3D) model of a heart. Methods may include determining, based on at least the at least one 3D model, an endocardial surface of the heart. Methods may include determining, based on at least the at least one 3D model, an epicardial surface of the heart. Methods may include causing a vector to be projected from the catheter tip. The vector may intersect the endocardial surface at a first point. The vector may intersect the epicardial surface at a second point. Methods may include determining, using at least the first point and the second point, a tissue thickness. Methods may include causing, via a display, output of an indication of the tissue thickness.

## Description

### BACKGROUND

Tissue, such as heart tissue, may vary in thickness from patient to patient. For some procedures, such as ventricular tachycardia ablation, a tissue thickness of the patient is helpful for planning and executing the procedures. Knowledge of the tissue thickness would be helpful for effectiveness and safety of the procedures. However, the tissue thickness is difficult to accurately ascertain.

Improvements are needed.

### SUMMARY

The following summary is for illustrative purposes only, and is not intended to limit or constrain the detailed description.

The present disclosure generally relates to systems and methods for auto-detecting (e.g., auto-defining, etc.) endocardial and epicardial surfaces in real-time. The present disclosure relates to systems and methods for determining and displaying a thickness of tissue between the endocardial and epicardial surfaces. The present disclosure generally relates to systems and methods tracking a thickness of tissue in front of an ablation catheter in real-time. The present disclosure relates to tracking of a device such as an imaging device and/or the ablation catheter in real-time in order to detect the tissue thickness.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

Methods may include receiving, at a computing device, location information associated with a catheter tip disposed within a heart of a subject. Methods may include receiving, at the computing device, imaging information. At least a portion of the imaging information may include first image data indicative of at least a portion of an endocardial surface of the heart. The at least a portion of the image information may include second image data indicative of at least a portion of an epicardial surface of the heart. Methods may include determining, based on at least the first image data, a first distance indicative of a distance from the catheter tip to the at least the portion of the endocardial surface in a first direction. Methods may include determining, based on at least the second image data, a second distance indicative of a distance from the catheter tip to the at least the portion of the epicardial surface in the first direction. Methods may include determining a tissue thickness based at least on the first distance and the second distance. Methods may include causing, via a display, output of thickness information indicative of the tissue thickness. Other embodiments of this aspect include corresponding computer systems, apparatus, and computer programs recorded on one or more computer storage devices, each configured to perform the actions of the methods.

Methods may include receiving, at a computing device, at least one coordinate set associated with a catheter tip. Methods may include receiving, at the computing device, at least one three-dimensional (3D) model of a heart. Methods may include determining, based on at least the at least one 3D model, an endocardial surface of the heart. Methods may include determining, based on at least the at least one 3D model, an epicardial surface of the heart. Methods may include causing a vector to be projected from the catheter tip. The vector may intersect the endocardial surface at a first point. The vector may intersect the epicardial surface at a second point. Methods may include determining, using at least the first point and the second point, a tissue thickness. Methods may include causing, via a display, output of an indication of the tissue thickness. Other embodiments of this aspect include corresponding computer systems, apparatus, and computer programs recorded on one or more computer storage devices, each configured to perform the actions of the methods.

Methods may include receiving, at a computing device, at least one coordinate set associated with a catheter tip. Methods may include receiving, at the computing device, at least one three-dimensional (3D) model of a heart. Methods may include determining, based on the at least one 3D model, an endocardial surface of the heart. Methods may include determining, based on the at least one 3D model, an epicardial surface of the heart. Methods may include causing a first plane to be created in the at least one 3D model of the heart. The first plane may be associated with the endocardial surface. Methods may include causing a second plane to be created in the at least one 3D model of the heart. The second plane may be associated with the epicardial surface. The first plane may be parallel to the second plane. Methods may include determining a first point on the first plane. The first point may be a closest point on the first plane to the catheter tip. Methods may include causing a vector to extend from the catheter tip through the first point and to a second point on the second plane. Methods may include determining a tissue thickness based at least on a distance along the vector from the first point to the second point. Methods may include causing, via a display, output indicative of the tissue thickness. Other embodiments of this aspect include corresponding computer systems, apparatus, and computer programs recorded on one or more computer storage devices, each configured to perform the actions of the methods.

Systems may include one or more processors configured to: receive, at a computing device, location information associated with a catheter tip disposed within a heart of a subject; receive, at the computing device, imaging information, where at least a portion of the imaging information may include first image data indicative of at least a portion of an endocardial surface of the heart, and where the at least a portion of the image information may include second image data indicative of at least a portion of an epicardial surface of the heart; determine, based on at least the first image data, a first distance indicative of a distance from the catheter tip to the at least the portion of the endocardial surface in a first direction; determine, based on at least the second image data, a second distance indicative of a distance from the catheter tip to the at least the portion of the epicardial surface in the first direction; determine a tissue thickness based at least on the first distance and the second distance; and cause, via a display, output of thickness information indicative of the tissue thickness. Other embodiments of this aspect include corresponding computer systems, apparatus, and computer programs recorded on one or more computer storage devices, each configured to perform the actions of the methods.

Systems may include one or more processors configured to: receive, at a computing device, at least one coordinate set associated with a catheter tip; receive, at the computing device, at least one three-dimensional (3D) model of a heart; determine, based on at least the at least one 3D model, an endocardial surface of the heart; determine, based on at least the at least one 3D model, an epicardial surface of the heart; cause a vector to be projected from the catheter tip, where the vector intersects the endocardial surface at a first point, and where the vector intersects the epicardial surface at a second point; determine, using at least the first point and the second point, a tissue thickness; and cause, via a display, output of an indication of the tissue thickness. Other embodiments of this aspect include corresponding computer systems, apparatus, and computer programs recorded on one or more computer storage devices, each configured to perform the actions of the methods.

Systems may include one or more processors configured to: receive, at a computing device, at least one coordinate set associated with a catheter tip; receive, at the computing device, at least one three-dimensional (3D) model of a heart; determine, based on the at least one 3D model, an endocardial surface of the heart; determine, based on the at least one 3D model, an epicardial surface of the heart; cause a first plane to be created in the at least one 3D model of the heart, where the first plane is associated with the endocardial surface; cause a second plane to be created in the at least one 3D model of the heart, where the second plane is associated with the epicardial surface, and where the first plane is parallel to the second plane; determine a first point on the first plane, where the first point is a closest point on the first plane to the catheter tip; cause a vector to extend from the catheter tip through the first point and to a second point on the second plane; determine a tissue thickness based at least on a distance along the vector from the first point to the second point; and cause, via a display, output indicative of the tissue thickness. Other embodiments of this aspect include corresponding computer systems, apparatus, and computer programs recorded on one or more computer storage devices, each configured to perform the actions of the methods.

These and other features and advantages are described in greater detail below.

### BRIEF DESCRIPTION OF THE DRAWINGS

The present disclosure is pointed out with particularity in the appended claims. However, other features of the present disclosure will become more apparent and the present disclosure will be best understood by referring to the following detailed description in conjunction with the accompany drawings in which:
Fig. 1 is a schematic, pictorial illustration of a catheter-based ultrasound imaging system using a catheter with a distal end assembly comprising an ultrasound array and a position sensor, in accordance with an embodiment of the present invention;
Fig. 2 shows example left ventricle images according to the systems and methods described herein.
Fig. 3 shows an example ventricle map according to the systems and methods described herein.
Fig. 4 shows an example output according to the systems and methods described herein.
Fig. 5 shows a flow diagram of an example method described herein.
Fig. 6 shows a flow diagram of an example method described herein.
Figs. 7A and 7B show a flow diagram of an example method described herein.

The accompanying drawings show examples of the disclosure. It is to be understood that the examples shown in the drawings and/or discussed herein are non-exclusive and that there are other examples of how the disclosure may be practiced.

### DETAILED DESCRIPTION

The present disclosure relates to a system and method for tracking and displaying heart tissue thickness.

The accompanying drawings, which form a part hereof, show examples of the disclosure. It is to be understood that the examples shown in the drawings and/or discussed herein are non-exclusive and that there are other examples of how the disclosure may be practiced.

The present disclosure relates to an algorithm designed to automatically measure and display tissue thickness in real-time during cardiac ablation procedures, using a position of a catheter tip within a target anatomical structure, such as a heart. The present disclosure ensure efficacy and safety of a procedure, particularly in complex cases such as ventricular tachycardia (VT) where tissue thickness can vary significantly.

The present disclosure may comprise receiving input data. Input data may comprise catheter position data, ultrasound imaging data, etc. Catheter position data may comprise a three-dimensional (3D) position of a catheter tip. The catheter position data may comprise a normal vector at the catheter tip position. The normal vector may represent a projection direction. The 3D position of the catheter tip may be continuously tracked using an ultrasound-based tracking system. The tracking system may provide X, Y, and Z coordinates of the catheter tip in real-time. Ultrasound imaging data may comprise high-resolution ultrasound images. Ultrasound imaging data may comprise two-dimensional (2D) images. Ultrasound imaging data may provide detailed views of endocardial (inner) and epicardial (outer) surfaces of heart tissue. Ultrasound imaging data may comprise a 3D model of the tissue, represented by the endocardial and/or epicardial surfaces.

The present disclosure relates to an algorithm comprising a surface detection component, a projection and measurement component, and a real-time display component. The surface detection component may comprise an endocardial surface identification component and an epicardial surface identification component. The endocardial surface identification component may comprise processing ultrasound images to identify and delineate the endocardial surface in the ultrasound images. The ultrasound images may be the 2D images. The endocardial surface identification component may use one or more edge detection technique. The endocardial surface identification may use one or more machine learning model trained to recognize characteristic patterns of an endocardial boundary.

The epicardial surface identification component may comprise processing ultrasound images to identify and delineate the epicardial surface in the ultrasound images. The epicardial surface identification component may use one or more edge detection technique. The epicardial surface identification may use one or more machine learning model trained to recognize characteristic patterns of an epicardial boundary.

The surface detection component may create a first simple plane representing the endocardial surface and a second simple plane representing the epicardial boundary, wherein the first plane is parallel to the second plane.

The projection and measurement component may comprise a projection calculation component. The projection calculation component may cause a projection line to be drawn from location in an image representative of a catheter tip perpendicular to a detected endocardial surface. The projection calculation component may cause the projection line to be extended to a detected epicardial surface. The projection calculation component may cause the portion of the projection line extending between the detected endocardial surface and the detect epicardial surface to represent a thickness of tissue between the detected endocardial surface and the detect epicardial surface. The portion of the projection line extending between the detected endocardial surface and the detect epicardial surface may form a straight-line distance between the detected endocardial surface and the detect epicardial surface. The projection calculation component may project a normal vector from the catheter tip onto the endocardial surface at a first project point and the epicardial surface at a second projection point. The projection calculation component and/or the distance measurement component may calculate a distance between the first projection point and the second projection point to determine a tissue thickness.

The projection and measurement component may comprise a distance measurement component. The distance measurement component may determine a length associated with the portion of the projection line extending between the detected endocardial surface and the detect epicardial surface. The distance measurement component may determine a length between the first projection point and the second projection point. The distance measurement component may compute distances in real-time. The distance measurement component may convert distances to millimeters (mm). The distance measurement component may provide a precise measurement of tissue thickness.

The projection and measurement component may find a closest point on a surface of the first plane and/or the second plane to the catheter tip and project a normal vector from the catheter tip to the closest point. The projection and measurement component may determine a distance on the normal vector between a first point where the normal vector intersects the first plane and a second point where the normal vector intersects the second plane.

The real-time display component may comprise a thickness display component. The thickness display component may display a calculated thickness, such as a thickness calculated by the distance measurement component, in real-time on a system interface, such as a display, monitor, etc. The thickness display component may display the calculated thickness adjacent to a location of a catheter tip. The thickness display component may cause the displayed calculated thickness to be continuously updated as a catheter moves. The thickness display component may ensure that an operator has accurate and up-to-date information regarding tissue thickness adjacent to the location of the catheter tip.

The real-time display component may comprise a color-coding component. The color-coding component may enhance visualization. The color-coding component may cause tissue thickness to be color-coded based on predefined thresholds. For example, tissue displayed as green may comprise a thickness satisfying a safe thickness threshold, tissue displayed as red may comprise a thickness not satisfying the safe thickness threshold, etc. The color-coding component may allow for quick assessment of tissue conditions during a procedure. The real-time display component may display a catheter tip, a tissue model, and/or a thickness measurement in a 3D model.

The real-time display component may plot the endocardial surface and/or first plane, the epicardial surface and/or second plane, the catheter tip, the projection points, the normal vector, and/or the calculated tissue thickness in a 3D plot.

The present disclosure relates to an error handling component. The error handling component may comprise a boundary detection confidence component. The boundary detection confidence component may determine a confidence level for a boundary detected. The boundary detection confidence component may compare the confidence level to a confidence level threshold. The boundary detection confidence component may generate an alert in response to the confidence level failing to satisfy the confidence level threshold. The confidence level may be affected by factors such as image quality, etc. The boundary detection confidence component may flag an associated thickness measurement as potentially inaccurate.

The error handling component may comprise a fallback mechanism component. The fallback mechanism may prompt an operator to reposition a catheter for a better angle. The fallback mechanism may revert to using a last known good measurement. If the epicardial surface cannot be reliably detected, then the fallback mechanism may revert to using the last known good measurement of the epicardial surface and/or prompt the operator to reposition the catheter for a better angle for the epicardial surface.

The present disclosure relates to a calibration component. The calibration component may comprise an initial calibration using an object with a known thickness, such as a phantom model or cadaver study, to ensure accuracy. The calibration component may adjust projection and/or measurement parameters to match real-world dimensions.

The present disclosure relates to a validation component. The validation component may validate accuracy of output, such as thickness, determined herein. The validation component may compare output determined herein with ground truth data obtained from a computed tomograpy (CT) scan or other high-precision imaging modality. The validation component may comprise performing statistical analysis to determine that determinations made herein are within acceptable error margins.

The systems and/or methods described herein may be used with a CARTO^{®} System. The systems and/or methods described herein may be fully integrated with the CARTO^{®} System. The systems and/or methods described herein may utilize real-time data feeds and/or a user interface of the CARTO^{®} System. The tissue thickness measurements obtained using the systems and/or methods described herein may be available as an additional layer of information on CARTO^{®} maps, aiding in the planning and execution of an ablation procedure.

In addition to ultrasound imaging data, the tissue tracking systems and/or methods described herein may be implemented with other imaging data, such as computer tomography (CT) imaging data, for example.

It is to be understood that both the following general description and the following detailed description are exemplary and explanatory only and are not restrictive.

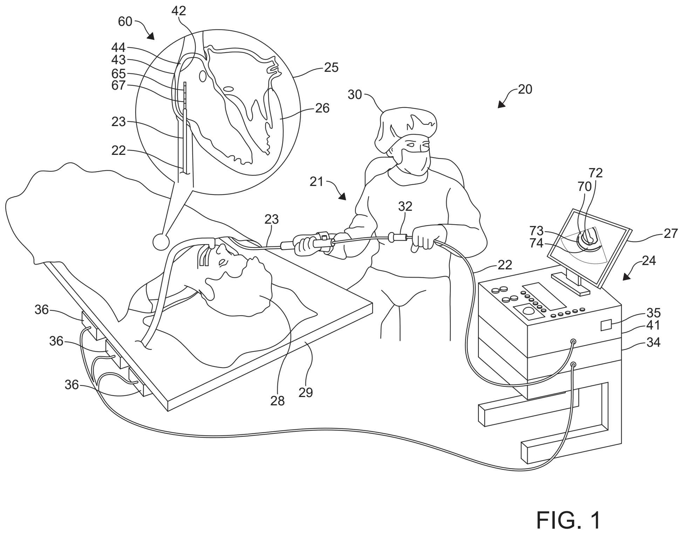

FIG. 1 is a schematic, pictorial illustration of a catheter-based ultrasound imaging system 20 using a catheter 21 with a distal end assembly 60 (shown in an inset 25) comprising an ultrasound array (transducer array) 65 and position sensor 67. The ultrasound array 65 may generate ultrasound images. The position sensor 67 may be preregistered with the ultrasound array 65 of the catheter 21.

The position sensor 67 may be configured to output signals indicative of a location and orientation of the ultrasound array 65 inside the organ. A processor (computing device, etc.) 41 of the system 20 may be configured to use the sensor's signal output to acquire one or more ultrasound images of anatomical structures oriented in various respective orientations relative to ultrasound array 65.

As seen, the distal end assembly 60 may be fitted at the distal end of a shaft 22 of the catheter 21. The shaft 22 may be inserted through a sheath 23 into a heart 26 of a patient 28 lying on a surgical table 29. The proximal end of shaft 22 may be connected to a control console 24.

A physician 30 may navigate the distal end assembly (tip, etc.) 60 of the catheter 21 into the heart 26 of the patient 28 to prepare for and/or perform an ablation procedure. To rotate the ultrasound array 65 into a required orientation, the physician 30 can, for example, use a manipulator 32 near the proximal end of the catheter 21. The heart 26 may comprise an endocardial (inner, etc.) surface 42, an epicardial (outer, etc.) surface 43, and tissue 44 between the endocardial surface 42 and the epicardial surface 43.

The control console 24 may comprise the processor (computing device, etc.) 41, typically a general-purpose computer, with suitable front end and interface circuits 38 for receiving signals from catheter 21. The console 24 may comprise a driver circuit 34 configured to drive magnetic field generators 36. During the navigation of the distal end 22 in the heart 26, the console 24 may receive location and orientation signals from the position sensor 67 in response to magnetic fields from external field generators 36. Magnetic field generators 36 may be placed at known positions external to patient 28, e.g., below table 29 upon which the patient 28 is lying. These location and orientation signals may be indicative of the location and orientation of ultrasound-array 65 in a coordinate system of the position tracking system.

The method of location and orientation sensing using external magnetic fields may be implemented in various medical applications, for example, in the CARTO^{™} system, produced by Biosense Webster, and is described in detail in U.S. Pat. Nos. 6,618,612 and 6,332,089, in PCT Patent Publication WO 96/05768, and in U.S. Patent Application Publications 2002/0065455, 2003/0120150, and 2004/0068178, whose disclosures are all incorporated herein by reference.

An imaged target anatomical structure may be presented to the physician 30 by the processor 41 on a monitor (display, etc.) 27, e.g., as a visual representation of the heart 70. The visual representation of the heart 70 may comprise an indication of the endocardial surface 72, an indication of the epicardial surface 73, and/or an indication of tissue between the endocardial surface and the epicardial surface 74. The indication of the endocardial surface 72 may correspond with the endocardial surface 42. The indication of the epicardial surface 73 may correspond with the epicardial surface 73. The indication of the tissue between the endocardial surface and the epicardial surface 74 may correspond with the tissue 44 between the endocardial surface 42 and the epicardial surface 43.

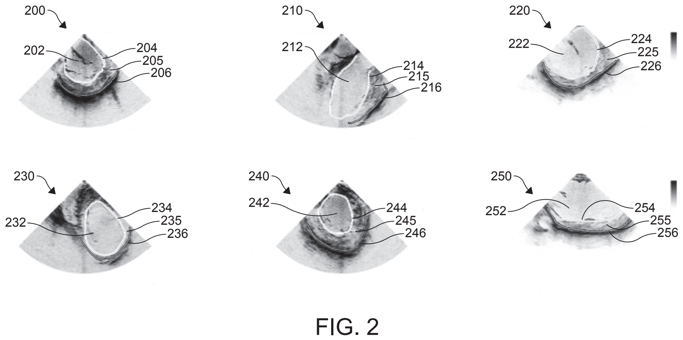

The monitor 27 may comprise one or more colors associated with the indication of the endocardial surface 72, the indication of the epicardial surface 73, and/or the indication of tissue between the endocardial surface and the epicardial surface 74. The monitor 27 may display a thickness indicator of the tissue 44 between the endocardial surface 42 and the epicardial surface 43. The thickness indicator may comprise one or more numbers, such as a number of millimeters of thickness. The thickness indicator may comprise one or more colors, such as green where thickness associated with the tissue 44 is above a thickness threshold and red where thickness associated with the tissue is below the thickness threshold. Figs. 2, 3, and 4 show examples of what may be shown on the monitor 27 in systems and methods described herein.

In the embodiment described herein, the catheter 21 may be used for ultrasound-based diagnostic purposes and further used to perform electrical sensing and/or ablation of tissue, such as tissue 44, in the heart 26, using, for example, one or more electrodes (not shown) disposed on the distal end.

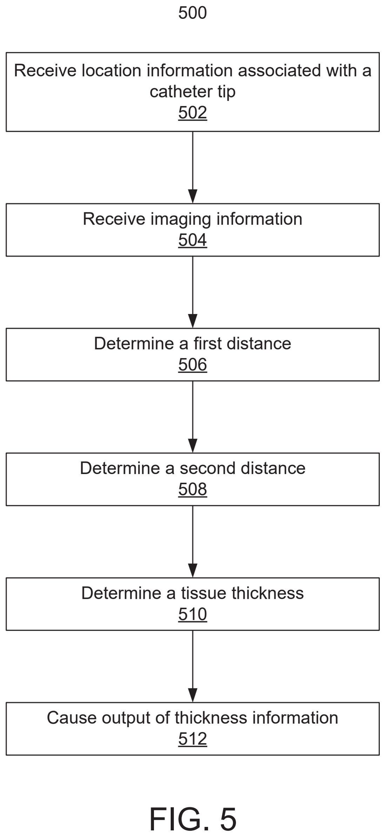

The processor 41 may be programmed in software to carry out the functions described herein. The software may be downloaded to a memory 35 of the computer in electronic form, over a network, for example, or it may, alternatively or additionally, be provided and/or stored on non-transitory tangible media, such as magnetic, optical, or electronic memory. In particular, the processor 41 may run one or more algorithms as disclosed herein, including in Figs. 5-7, that enables processor 41 to perform the disclosed steps, as further described below.

The example configuration shown in FIG. 1 is chosen purely for the sake of conceptual clarity. The disclosed techniques may similarly be applied using other contexts where knowledge of heart tissue thickness would be useful.

The processor 41 may receive location information associated with the distal end assembly 60 of the catheter 21 originating from the position sensor 67 in the heart 26 of a patient 28. The processor 41 may receive ultrasound imaging information from the ultrasound array 65. The ultrasound imaging information may comprise at least a portion of the endocardial surface 42. The ultrasound imaging information may comprise at least a portion of the epicardial surface 43. The processor 41 may determine a first distance from the distal end assembly 60 of the catheter 21 to the at least a portion of the endocardial surface 42 in a first direction. The processor 41 may determine a second distance from the distal end assembly 60 of the catheter 21 to the at least a portion of the epicardial surface 43 in the first direction. The processor 41 may determine a thickness of the tissue 44 between the endocardial surface 42 and the epicardial surface 43. The monitor 27 may output the thickness of the tissue 44 between the endocardial surface 42 and the epicardial surface 43. The monitor 27 may display the visual representation of the heart 70, the indication of the endocardial surface 72, the indication of the epicardial surface 73, and/or the indication of tissue between the endocardial surface and the epicardial surface 74.

The processor 41 may receive at least one coordinate set associated with the distal end assembly 60 of the catheter 21 based on one or more signals originating from the position sensor 67 in the heart 26 of a patient 28. The processor 41 may receive at least one three-dimensional (3D) model based on one or more signals originating from the ultrasound array 65. The visual representation of the heart 70 may comprise the at least one 3D model. The processor 41 may determine the endocardial surface 42 of the heart 26 based on at least the at least one 3D model. The processor 41 may determine the epicardial surface 43 of the heart 26 based on at least the at least one 3D model. The processor 41 may cause a vector to be projected on or over the 3D model (or other output). The vector may extend from a location associated with the distal end assembly 60 of the catheter 21. The vector may intersect the endocardial surface 42 at a first point. The vector may intersect the epicardial surface 43 at a second point. The processor 41 may determine a thickness of the tissue 44 in between the endocardial surface 42 and the epicardial surface 43 using at least the first point and the second point. The processor 41 may cause the monitor 27 to output an indication of the tissue 44 thickness.

The processor 41 may receive at least one coordinate set associated with the distal end assembly 60 of the catheter 21 based on one or more signals originating from the position sensor 67 in the heart 26 of a patient 28. The processor 41 may receive at least one three-dimensional (3D) model based on one or more signals originating from the ultrasound array 65. The visual representation of the heart 70 may comprise the at least one 3D model. The processor 41 may determine the endocardial surface 42 of the heart 26 based on at least the at least one 3D model. The processor 41 may determine the epicardial surface 43 of the heart 26 based on at least the at least one 3D model. The processor 41 may cause a first plane to be created in the at least one 3D model.

The first plane may be associated with the endocardial surface 42. The processor 41 may cause a second plane to be created in the at least one 3D model. The second plane may be associated with the epicardial surface 43. The first plane may be parallel to the second plane. The processor 41 may determine a first point on the first plane. The first point may be a closest point on the first plane to the distal end assembly 60 of the catheter 21. The processor 41 may cause a vector to extend from a location associated with the distal end assembly 60 of the catheter 21 to a second point on the second plane. The processor 41 may determine a tissue 44 thickness based at least on a distance along the vector from the first point to the second point. The processor 41 may cause output indicative of the tissue 44 thickness on the monitor 27.

Although Fig. 1 illustrates the tissue tracking systems and/or methods described herein implemented with ultrasound imaging information, the tissue tracking systems and/or methods described herein may be implemented with other imaging data, such as computer tomography (CT) imaging data, for example.

Fig. 2 shows example left ventricle images 200, 210, 220, 230, 240, 250 according to the systems and methods described herein. The images 200, 210, 220, 230, 240, 250 may show a cavity, such as cavity 202 in image 200, cavity 212 in image 210, cavity 222 in image 220, cavity 232 in image 230, cavity 242 in image 240, and cavity 252 in image 250. An imaging catheter may take an image from the cavities 202, 212, 222, 232, 242, 252 depicted in the images 200, 210, 220, 230, 240, 250.

The images 200, 210, 220, 230, 240, 250 may show an endocardial surface, such as endocardial surface 204 in image 200, endocardial surface 214 in image 210, endocardial surface 224 in image 220, endocardial surface 234 in image 230, endocardial surface 244 in image 240, and endocardial surface 254 in image 250. The systems and methods described herein may cause the endocardial surfaces 204, 214, 224, 234, 244, 254 to be highlighted (color coded, bolded, etc.) in the images 200, 210, 220, 230, 240, 250.

The images 200, 210, 220, 230, 240, 250 may show an epicardial surface, such as epicardial surface 206 in image 200, epicardial surface 216 in image 210, epicardial surface 226 in image 220, epicardial surface 236 in image 230, epicardial surface 246 in image 240, and epicardial surface 256 in image 250. The systems and methods described herein may cause the epicardial surfaces 206, 216, 226, 236, 246, 256 to be highlighted (color coded, bolded, etc.) in the images 200, 210, 220, 230, 240, 250.

The images 200, 210, 220, 230, 240, 250 may show tissue between the endocardial surface and the epicardial surface, such as tissue 205 in image 200, tissue 215 in image 210, tissue 225 in image 220, tissue 235 in image 230, tissue 245 in image 240, and tissue 255 in image 250. The systems and methods described herein may cause the tissue 205, 215, 225, 235, 245, 255 to be highlighted (color coded, bolded, etc.) in the images 200, 210, 220, 230, 240, 250. The systems and methods described herein may cause thickness of the tissue 205, 215, 225, 235, 245, 255 to be indicated in the images 200, 210, 220, 230, 240, 250.

Although the images 200, 210, 220, 230, 240, 250 depict left ventricles, other portions of the heart, or the entire heart may be imaged using the systems and methods described herein.

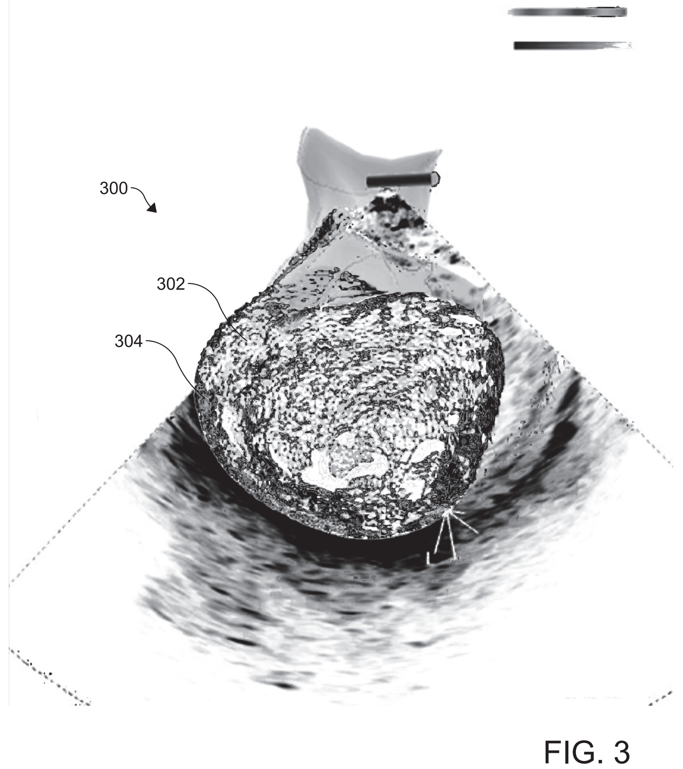

Fig. 3 shows an example ventricle map 300 according to the systems and methods described herein. The ventricle map 300 may depict tissue thickness. For example, tissue thickness may be depicted by color. Lighter areas, such as area 302 may represent areas of thinner tissue. Darker areas, such as area 304, may represent areas of thicker tissue. In an embodiment, lighter areas, such as area 302 may represent areas of thicker tissue, and darker areas, such as area 304, may represent areas of thinner tissue. In an embodiment, a particular color may correspond to a particular threshold and/or range. In an embodiment, two colors may be on either end of a spectrum, and a combination of the two colors indicates a thickness. For example, white may be associated with no tissue (or a minimum thickness value), black may be associated with a maximum thickness value, and a shade of gray at a particular spot in the ventricle map 300 may indicate a thickness, where the darker the color, the more thick the tissue.

Although the map 300 was a ventricle map, other portions of the heart, or the entire heart may be mapped using the systems and methods described herein.

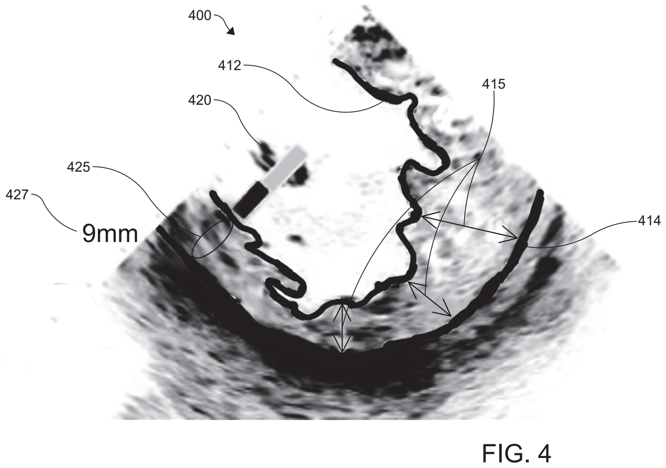

Fig. 4 shows an example output 400 according to the systems and methods described herein. The output 400 may comprise an image of at least part of a heart. The output 400 may comprise an indication of an endocardial surface 412. The output 400 may comprise an indication of an epicardial surface 414. The output 400 may comprise tissue 415 between the endocardial surface and the epicardial surface. The output 400 may comprise an indication of a catheter 420. The indication of the catheter 420 may depict a position and/or orientation of the catheter relative to the endocardial surface and/or the epicardial surface. The output 400 may comprise a focus 425. The focus 425 may indicate tissue between the endocardial surface and the epicardial surface in a direction of the catheter. The output may comprise a thickness value 427. The thickness value 427 may comprise a thickness of the tissue indicated by the focus 425. The catheter may comprise an ablation catheter. In another embodiment, a user may select an area of tissue from the output 400, and the area selected may be indicated by the focus 425 and the corresponding thickness may be shown on the thickness value 427. The focus 425 may comprise an indication of tissue of interest by applying a border around the tissue of interest, highlighting the tissue of interest, color coding, bolding, or any other known method of providing focus to the tissue on interest.

Fig. 5 is a flowchart of an example process 500. In some implementations, one or more process blocks of Fig. 5 may be performed by a computing device, such as the processor 41 in Fig. 1.

As shown in Fig. 5, process 500 may include receiving location information associated with a catheter tip (block 502). For example, the processor 41 may receive location information associated with a catheter tip. The catheter tip may be disposed within a heart of a subject. The location information comprises three-dimensional (3D) coordinate information. The catheter tip may be associated with an ablation catheter.

As also shown in Fig. 5, process 500 may include receiving imaging information (block 504). For example, the processor 41 may receive imaging information. At least a portion of the imaging information may include first image data indicative of at least a portion of an endocardial surface of the heart. The at least a portion of the image information may include second image data indicative of at least a portion of an epicardial surface of the heart. The imaging information may comprise one or more of ultrasound imaging information, computer tomography (CT) imaging information, etc.

As further shown in Fig. 5, process 500 may include determining a first distance (block 506). For example, the processor 41 may determine a first distance. The first distance may be determined based on at least the first image data. The first distance may be indicative of a distance from the catheter tip to the at least the portion of the endocardial surface in a first direction.

As also shown in Fig. 5, process 500 may include determining a second distance (block 508). For example, the processor 41 may determine a second distance. The second distance may be determined based on at least the second image data. The second distance may be indicative of a distance from the catheter tip to the at least the portion of the epicardial surface in the first direction. The first distance may be determined before the second distance is determined. The second distance may be determined before the first distance is determined. The at least the portion of the epicardial surface may be distinguished from additional layer data, such as a portion of a pericardial surface, using one or more distinguishing techniques. The one or more distinguishing techniques may comprise one or more of auto-contouring, machine learning image identification, manual image identification, etc.

As further shown in Fig. 5, process 500 may include determining a tissue thickness (block 510). For example, the processor 41 may determine a tissue thickness. The tissue thickness may be determined based at least on the first distance and the second distance. The tissue thickness may be determined by measuring from an endpoint associated with the first distance to an endpoint associated with the second distance. The tissue thickness may be determined by measuring from an endpoint associated with the second distance to an endpoint associated with the first distance. The process 500 may include determining a confidence associated with the tissue thickness. For example, the processor 41 may determine a confidence associated with the tissue thickness. The process 500 may include comparing the confidence to a confidence threshold. For example, the processor 41 may compare the confidence to a confidence threshold. The process 500 may include causing an alert to be generated in response to determining that the confidence does not satisfy the confidence threshold. For example, the processor 41 may cause an alert to be generated in response to determining that the confidence does not satisfy the confidence threshold. The process 500 may include causing an indication to be generated in response to determining that the confidence satisfies the confidence threshold. For example, the processor 41 may cause an indication to be generated in response to determining that the confidence satisfies the confidence threshold. U.S. Pat. No. 10,517,670, whose disclosure is incorporated herein by reference, describes an "ablation index," which gives a measure of the depth of an ablation lesion as an integral over the temporal duration of the product of the force raised to one non-unity exponent and the power raised to another non-unity exponent. The tissue thickness determined herein may be used to derive an appropriate ablation index.

As also shown in Fig. 5, process 500 may include causing output of thickness information (block 512). For example, the processor 41 may cause output of thickness information. The thickness information may be output via a display. The thickness information may be indicative of the tissue thickness. The output of the thickness information may be indicated by a first color. A first portion in the information indicative of the tissue thickness may be bounded by a first point and a second point. The first point may comprise a point where a hypothetical line from the catheter tip extending in the first direction touches the endocardial surface. The second point may comprise a point where the hypothetical line touches the epicardial surface. The first portion may comprise the hypothetical line between the first point and the second point. The first portion may comprise the first color. The tissue thickness may satisfy a thickness threshold and the first color may be green. The tissue thickness may not satisfy a thickness threshold and the first color may be red.

Although Fig. 5 shows example blocks of process 500, in some implementations, process 500 may include additional blocks, fewer blocks, different blocks, or differently arranged blocks than those depicted in Fig. 5. Additionally, or alternatively, two or more of the blocks of process 500 may be performed in parallel.

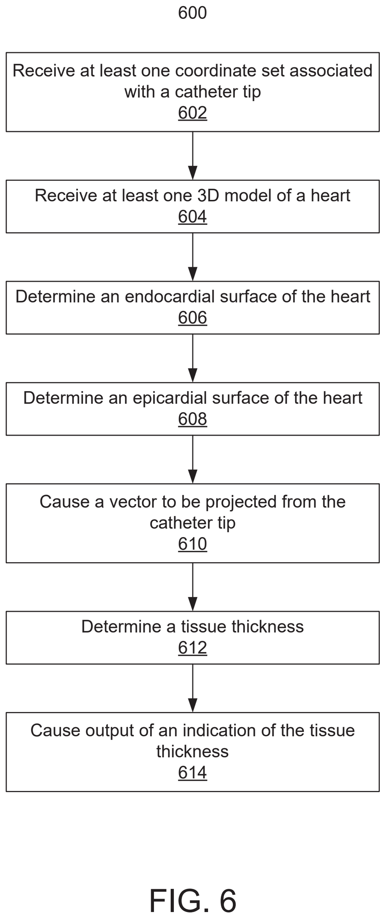

Fig. 6 is a flowchart of an example process 600. In some implementations, one or more process blocks of Fig. 6 may be performed by a computing device, such as the processor 41 in Fig. 1.

As shown in Fig. 6, process 600 may include receiving at least one coordinate set associated with a catheter tip (block 602). For example, the processor 41 may receive at least one coordinate set associated with a catheter tip. The catheter tip may be associated with an ablation catheter.

As also shown in Fig. 6, process 600 may include receiving at least one three-dimensional (3D) model of a heart (block 604). For example, the processor 41 may receive at least one three-dimensional (3D) model of a heart.

As further shown in Fig. 6, process 600 may include determining an endocardial surface of the heart (block 606). For example, the processor 41 may determine an endocardial surface of the heart. The endocardial surface of the heart may be determined based on at least the at least one 3D model. An indication of the endocardial surface may be caused to be created in the at least one 3D model. The indication may comprise a boldening, a highlighting, a color-coding, etc.

As also shown in Fig. 6, process 600 may include determining an epicardial surface of the heart (block 608). For example, the processor 41 may determine an epicardial surface of the heart. The epicardial surface of the heart may be determined based on at least the at least one 3D model. An indication of the epicardial surface may be caused to be created in the at least one 3D model. The indication may comprise a boldening, a highlighting, a color-coding, etc. The endocardial surface may be determined before the epicardial surface is determined. The epicardial surface may be determined before the endocardial surface is determined. The epicardial surface may be distinguished from additional layer data, such as a pericardial surface, using one or more distinguishing techniques. The one or more distinguishing techniques may comprise one or more of auto-contouring, machine learning image identification, manual image identification, etc.

As further shown in Fig. 6, process 600 may include causing a vector to be projected from the catheter tip (block 610). For example, the processor 41 may cause a vector to be projected from the catheter tip. The vector may intersect the endocardial surface at a first point. The vector may intersect the epicardial surface at a second point. The vector may be in the at least one 3D model.

As also shown in Fig. 6, process 600 may include determining a tissue thickness (block 612). For example, the processor 41 may determine a tissue thickness. The tissue thickness may be determined using at least the first point and the second point. The process 600 may include determining a confidence associated with the tissue thickness. For example, the processor 41 may determine a confidence associated with the tissue thickness. The process 600 may include comparing the confidence to a confidence threshold. For example, the processor 41 may compare the confidence to a confidence threshold. The process 600 may include causing an alert to be generated in response to determining that the confidence does not satisfy the confidence threshold. For example, the processor 41 may cause an alert to be generated in response to determining that the confidence does not satisfy the confidence threshold. The process 600 may include causing an indication to be generated in response to determining that the confidence satisfies the confidence threshold. For example, the processor 41 may cause an indication to be generated in response to determining that the confidence satisfies the confidence threshold. The tissue thickness may be determined by measuring from the first point to the second point. The tissue thickness may be determined by measuring from the second point to the first point. The tissue thickness determined herein may be used to derive an appropriate ablation index.

As further shown in Fig. 6, process 600 may include causing output of an indication of the tissue thickness (block 614). For example, the processor 41 may cause output of an indication of the tissue thickness. The indication of the tissue thickness may be output via a display. The indication of the tissue thickness may comprise a measurement. The indication of the tissue thickness may comprise a line along the vector from the first point to the second point. The line may comprise a color. The tissue thickness may satisfy a thickness threshold and the color may be green. The tissue thickness may not satisfy a thickness threshold and the color may be red. The process 600 may include causing, via the display, output of an indication of the catheter tip and the at least one 3D model. For example, the processor 41 may cause, via the display, output of an indication of the catheter tip and the at least one 3D model.

Although Fig. 6 shows example blocks of process 600, in some implementations, process 600 may include additional blocks, fewer blocks, different blocks, or differently arranged blocks than those depicted in Fig. 6. Additionally, or alternatively, two or more of the blocks of process 600 may be performed in parallel.

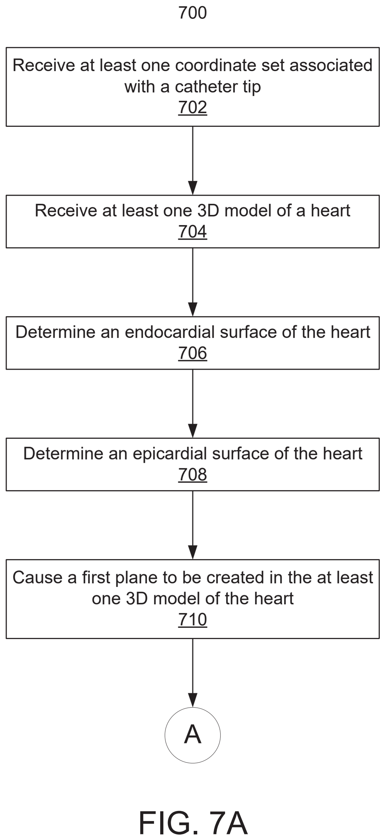

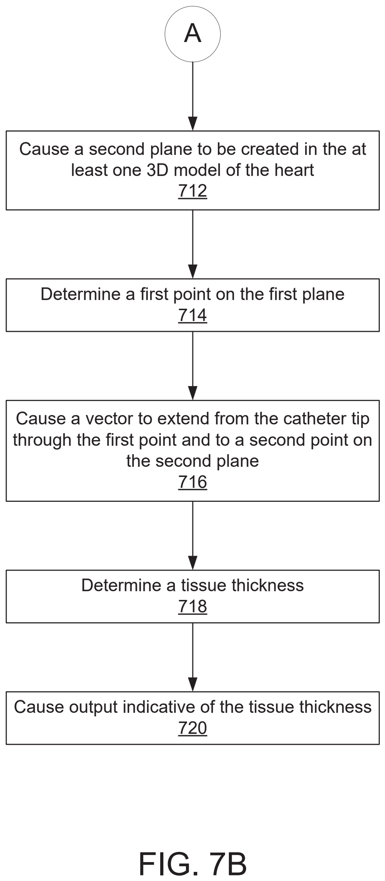

Figs. 7A and 7B show a flowchart of an example process 700. In some implementations, one or more process blocks of Figs. 7A and 7B may be performed by a computing device, such as the processor 41 in Fig. 1.

Process 700 may include receiving at least one coordinate set associated with a catheter tip (block 702). For example, the processor 41 may receive at least one coordinate set associated with a catheter tip. The catheter tip may be associated with an ablation catheter.

Process 700 may include receiving at least one three-dimensional (3D) model of a heart (block 704). For example, the processor 41 may receive at least one three-dimensional (3D) model of a heart.

Process 700 may include determining an endocardial surface of the heart (block 706). For example, the processor 41 may determine an endocardial surface of the heart. The endocardial surface of the heart may be determined based on the at least one 3D model. An indication of the endocardial surface may be caused to be created in the at least one 3D model. The indication may comprise a boldening, a highlighting, a color-coding, etc.

Process 700 may include determining an epicardial surface of the heart (block 708). For example, the processor 41 may determine an epicardial surface of the heart. The epicardial surface of the heart may be determined based on the at least one 3D model. An indication of the epicardial surface may be caused to be created in the at least one 3D model. The indication may comprise a boldening, a highlighting, a color-coding, etc. The endocardial surface may be determined before the epicardial surface is determined. The epicardial surface may be determined before the endocardial surface is determined. The epicardial surface may be distinguished from additional layer data, such as a pericardial surface, using one or more distinguishing techniques. The one or more distinguishing techniques may comprise one or more of auto-contouring, machine learning image identification, manual image identification, etc.

Process 700 may include causing a first plane to be created in the at least one 3D model of the heart (block 710). For example, the processor 41 may cause a first plane to be created in the at least one 3D model of the heart. The first plane may be associated with the endocardial surface.

Process 700 may include causing a second plane to be created in the at least one 3D model of the heart (block 712). For example, the processor 41 may cause a second plane to be created in the at least one 3D model of the heart. The second plane may be associated with the epicardial surface. The first plane may be parallel to the second plane.

Process 700 may include determining a first point on the first plane (block 714). For example, the processor 41 may determine a first point on the first plane. The first point may be a closest point on the first plane to the catheter tip.

Process 700 may include causing a vector to extend from the catheter tip through the first point and to a second point on the second plane (block 716). For example, the processor 41 may cause a vector to extend from the catheter tip through the first point and to a second point on the second plane. The vector may be in the at least one 3D model.

Process 700 may include determining a tissue thickness (block 718). For example, the processor 41 may determine a tissue thickness. The tissue thickness may be determined based at least on a distance along the vector from the first point to the second point. The process 700 may include determining a confidence associated with the tissue thickness. For example, the processor 41 may determine a confidence associated with the tissue thickness. The process 700 may include comparing the confidence to a confidence threshold. For example, the processor 41 may compare the confidence to a confidence threshold. The process 700 may include causing an alert to be generated in response to determining that the confidence does not satisfy the confidence threshold. For example, the processor 41 may cause an alert to be generated in response to determining that the confidence does not satisfy the confidence threshold. The process 700 may include causing an indication to be generated in response to determining that the confidence satisfies the confidence threshold. For example, the processor 41 may cause an indication to be generated in response to determining that the confidence satisfies the confidence threshold.

The tissue thickness may be determined by measuring from the first point to the second point. The tissue thickness may be determined by measuring from the second point to the first point. The tissue thickness determined herein may be used to derive an appropriate ablation index.

Process 700 may include causing output indicative of the tissue thickness (block 720). For example, the processor 41 may cause output indicative of the tissue thickness. The output indicative of the tissue thickness may be caused be presented on a display. The output indicative of the tissue thickness may comprise a measurement. The output indicative of the tissue thickness may comprise a line along the vector from the first point to the second point. The line may comprise a color. The tissue thickness may satisfy a thickness threshold and the color may be green. The tissue thickness may not satisfy a thickness threshold and the color may be red. The process 700 may include causing, via the display, output indicative of the endocardial surface, the epicardial surface, the catheter tip, the first point, and the second point. For example, the processor 41 may cause, via the display, output indicative of the endocardial surface, the epicardial surface, the catheter tip, the first point, and the second point.

Although Figs. 7A and 7B show example blocks of process 700, in some implementations, process 700 may include additional blocks, fewer blocks, different blocks, or differently arranged blocks than those depicted in Figs. 7A and 7B. Additionally, or alternatively, two or more of the blocks of process 700 may be performed in parallel.

### EXAMPLE CLAUSES

Example Clause 1: A method may include: receiving, at a computing device, location information associated with a catheter tip disposed within a heart of a subject; receiving, at the computing device, imaging information, where at least a portion of the imaging information may include first image data indicative of at least a portion of an endocardial surface of the heart, and where the at least a portion of the image information may include second image data indicative of at least a portion of an epicardial surface of the heart; determining, based on at least the first image data, a first distance indicative of a distance from the catheter tip to the at least the portion of the endocardial surface in a first direction; determining, based on at least the second image data, a second distance indicative of a distance from the catheter tip to the at least the portion of the epicardial surface in the first direction; determining a tissue thickness based at least on the first distance and the second distance; and causing, via a display, output of thickness information indicative of the tissue thickness.

Example Clause 2: The method of Example Clause 1, where the output of the thickness information is indicated by a first color.

Example Clause 3: The method of Example Clause 1 or Example Clause 2, where a first portion in the information indicative of the tissue thickness is bounded by a first point and a second point, where the first point may include a point where a hypothetical line from the catheter tip extending in the first direction touches the endocardial surface, where the second point may include a point where the hypothetical line touches the epicardial surface, where the first portion may include the hypothetical line between the first point and the second point, and where the first portion may include the first color.

Example Clause 4: The method of any one of Example Clauses 1-3, where the tissue thickness satisfies a thickness threshold and the first color is green.

Example Clause 5: The method of any one of Example Clauses 1-4, where the tissue thickness does not satisfy a thickness threshold and the first color is red.

Example Clause 6: The method of any one of Example Clauses 1-5, further may include determining a confidence associated with the tissue thickness.

Example Clause 7: The method of any one of Example Clauses 1-6, further may include: comparing the confidence to a confidence threshold; and causing an alert to be generated in response to determining that the confidence does not satisfy the confidence threshold.

Example Clause 8: The method of any one of Example Clauses 1-7, further may include: comparing the confidence to a confidence threshold; and causing an indication to be generated in response to determining that the confidence satisfies the confidence threshold.

Example Clause 9: The method of any one of Example Clauses 1-8, where the location information may include three-dimensional (3D) coordinate information.

Example Clause 10: A method may include: receiving, at a computing device, at least one coordinate set associated with a catheter tip; receiving, at the computing device, at least one three-dimensional (3D) model of a heart; determining, based on at least the at least one 3D model, an endocardial surface of the heart; determining, based on at least the at least one 3D model, an epicardial surface of the heart; causing a vector to be projected from the catheter tip, where the vector intersects the endocardial surface at a first point, and where the vector intersects the epicardial surface at a second point; determining, using at least the first point and the second point, a tissue thickness; and causing, via a display, output of an indication of the tissue thickness.

Example Clause 11: The method of Example Clause 10, where the indication of the tissue thickness may include a measurement.

Example Clause 12: The method of Example Clause 10 or Example Clause 11, where the indication of the tissue thickness may include a line along the vector from the first point to the second point.

Example Clause 13: The method of any one of Example Clauses 10-12, where the line may include a color.

Example Clause 14: The method of any one of Example Clauses 10-13, where the tissue thickness satisfies a thickness threshold and the color is green.

Example Clause 15: The method of any one of Example Clauses 10-14, where the tissue thickness does not satisfy a thickness threshold and the color is red.

Example Clause 16: The method of any one of Example Clauses 10-15, further may include causing, via the display, output of an indication of the catheter tip and the at least one 3D model.

Example Clause 17: The method of any one of Example Clauses 10-16, further may include determining a confidence associated with the tissue thickness.

Example Clause 18: The method of any one of Example Clauses 10-17, further may include: comparing the confidence to a confidence threshold; and causing an alert to be generated in response to determining that the confidence does not satisfy the confidence threshold.

Example Clause 19: The method of any one of Example Clauses 10-18, further may include: comparing the confidence to a confidence threshold; and causing an indication to be generated in response to determining that the confidence satisfies the confidence threshold.

Example Clause 20: A method may include: receiving, at a computing device, at least one coordinate set associated with a catheter tip; receiving, at the computing device, at least one three-dimensional (3D) model of a heart; determining, based on the at least one 3D model, an endocardial surface of the heart; determining, based on the at least one 3D model, an epicardial surface of the heart; causing a first plane to be created in the at least one 3D model of the heart, where the first plane is associated with the endocardial surface; causing a second plane to be created in the at least one 3D model of the heart, where the second plane is associated with the epicardial surface, and where the first plane is parallel to the second plane; determining a first point on the first plane, where the first point is a closest point on the first plane to the catheter tip; causing a vector to extend from the catheter tip through the first point and to a second point on the second plane; determining a tissue thickness based at least on a distance along the vector from the first point to the second point; and causing, via a display, output indicative of the tissue thickness.

Example Clause 21: The method of Example Clause 20, where the output indicative of the tissue thickness may include a measurement.

Example Clause 22: The method of Example Clause 20 or Example Clause 21, where the output indicative of the tissue thickness may include a line along the vector from the first point to the second point.

Example Clause 23: The method of any one of Example Clauses 20-22, where the line may include a color.

Example Clause 24: The method of any one of Example Clauses 20-23, where the tissue thickness satisfies a thickness threshold and the color is green.

Example Clause 25: The method of any one of Example Clauses 20-24, where the tissue thickness does not satisfy a thickness threshold and the color is red.

Example Clause 26: The method of any one of Example Clauses 20-25, further may include determining a confidence associated with the tissue thickness.

Example Clause 27: The method of any one of Example Clauses 20-26, further may include: comparing the confidence to a confidence threshold; and causing an alert to be generated in response to determining that the confidence does not satisfy the confidence threshold.

Example Clause 28: The method of any one of Example Clauses 20-27, further may include: comparing the confidence to a confidence threshold; and causing an indication to be generated in response to determining that the confidence satisfies the confidence threshold.

Example Clause 29: The method of any one of Example Clauses 20-28, further may include causing, via the display, output indicative of the endocardial surface, the epicardial surface, the catheter tip, the first point, and the second point.

Example Clause 30: A system may include: one or more processors configured to: receive, at a computing device, location information associated with a catheter tip disposed within a heart of a subject; receive, at the computing device, imaging information, where at least a portion of the imaging information may include first image data indicative of at least a portion of an endocardial surface of the heart, and where the at least a portion of the image information may include second image data indicative of at least a portion of an epicardial surface of the heart; determine, based on at least the first image data, a first distance indicative of a distance from the catheter tip to the at least the portion of the endocardial surface in a first direction; determine, based on at least the second image data, a second distance indicative of a distance from the catheter tip to the at least the portion of the epicardial surface in the first direction; determine a tissue thickness based at least on the first distance and the second distance; and cause, via a display, output of thickness information indicative of the tissue thickness.

Example Clause 31: The system of Example Clause 30, where the output of the thickness information is indicated by a first color.

Example Clause 32: The system of Example Clause 30 or Example Clause 31, where a first portion in the information indicative of the tissue thickness is bounded by a first point and a second point, where the first point may include a point where a hypothetical line from the catheter tip extending in the first direction touches the endocardial surface, where the second point may include a point where the hypothetical line touches the epicardial surface, where the first portion may include the hypothetical line between the first point and the second point, and where the first portion may include the first color.

Example Clause 33: The system of any one of Example Clauses 30-32, where the tissue thickness satisfies a thickness threshold and the first color is green.

Example Clause 34: The system of any one of Example Clauses 30-33, where the tissue thickness does not satisfy a thickness threshold and the first color is red.

Example Clause 35: The system of any one of Example Clauses 30-34, further may include determining a confidence associated with the tissue thickness.

Example Clause 36: The system of any one of Example Clauses 30-35, further may include: comparing the confidence to a confidence threshold; and causing an alert to be generated in response to determining that the confidence does not satisfy the confidence threshold.

Example Clause 37: The system of any one of Example Clauses 30-36, further may include: comparing the confidence to a confidence threshold; and causing an indication to be generated in response to determining that the confidence satisfies the confidence threshold.

Example Clause 38: The system of any one of Example Clauses 30-37, where the location information may include three-dimensional (3D) coordinate information.

Example Clause 39: A system may include: one or more processors configured to: receive, at a computing device, at least one coordinate set associated with a catheter tip; receive, at the computing device, at least one three-dimensional (3D) model of a heart; determine, based on at least the at least one 3D model, an endocardial surface of the heart; determine, based on at least the at least one 3D model, an epicardial surface of the heart; cause a vector to be projected from the catheter tip, where the vector intersects the endocardial surface at a first point, and where the vector intersects the epicardial surface at a second point; determine, using at least the first point and the second point, a tissue thickness; and cause, via a display, output of an indication of the tissue thickness.

Example Clause 40: The system of Example Clause 39, where the indication of the tissue thickness may include a measurement.

Example Clause 41: The system of Example Clause 39 or Example Clause 40, where the indication of the tissue thickness may include a line along the vector from the first point to the second point.

Example Clause 42: The system of any one of Example Clauses 39-41, where the line may include a color.

Example Clause 43: The system of any one of Example Clauses 39-42, where the tissue thickness satisfies a thickness threshold and the color is green.

Example Clause 44: The system of any one of Example Clauses 39-43, where the tissue thickness does not satisfy a thickness threshold and the color is red.

Example Clause 45: The system of any one of Example Clauses 39-44, further may include causing, via the display, output of an indication of the catheter tip and the at least one 3D model.

Example Clause 46: The system of any one of Example Clauses 39-45, further may include determining a confidence associated with the tissue thickness.

Example Clause 47: The system of any one of Example Clauses 39-46, further may include: comparing the confidence to a confidence threshold; and causing an alert to be generated in response to determining that the confidence does not satisfy the confidence threshold.

Example Clause 48: The system of any one of Example Clauses 39-47, further may include: comparing the confidence to a confidence threshold; and causing an indication to be generated in response to determining that the confidence satisfies the confidence threshold.

Example Clause 49: A system may include: one or more processors configured to: receive, at a computing device, at least one coordinate set associated with a catheter tip; receive, at the computing device, at least one three-dimensional (3D) model of a heart; determine, based on the at least one 3D model, an endocardial surface of the heart; determine, based on the at least one 3D model, an epicardial surface of the heart; cause a first plane to be created in the at least one 3D model of the heart, where the first plane is associated with the endocardial surface; cause a second plane to be created in the at least one 3D model of the heart, where the second plane is associated with the epicardial surface, and where the first plane is parallel to the second plane; determine a first point on the first plane, where the first point is a closest point on the first plane to the catheter tip; cause a vector to extend from the catheter tip through the first point and to a second point on the second plane; determine a tissue thickness based at least on a distance along the vector from the first point to the second point; and cause, via a display, output indicative of the tissue thickness.

Example Clause 50: The system of Example Clause 49, where the output indicative of the tissue thickness may include a measurement.

Example Clause 51: The system of Example Clause 49 or Example Clause 50, where the output indicative of the tissue thickness may include a line along the vector from the first point to the second point.

Example Clause 52: The system of any one of Example Clauses 49-51, where the line may include a color.

Example Clause 53: The system of any one of Example Clauses 49-52, where the tissue thickness satisfies a thickness threshold and the color is green.

Example Clause 54: The system of any one of Example Clauses 49-53, where the tissue thickness does not satisfy a thickness threshold and the color is red.

Example Clause 55: The system of any one of Example Clauses 49-54, further may include determining a confidence associated with the tissue thickness.

Example Clause 56: The system of any one of Example Clauses 49-55, further may include: comparing the confidence to a confidence threshold; and causing an alert to be generated in response to determining that the confidence does not satisfy the confidence threshold.

Example Clause 57: The system of any one of Example Clauses 49-56, further may include: comparing the confidence to a confidence threshold; and causing an indication to be generated in response to determining that the confidence satisfies the confidence threshold.

Example Clause 58: The system of any one of Example Clauses 49-57, further may include causing, via the display, output indicative of the endocardial surface, the epicardial surface, the catheter tip, the first point, and the second point.

Example Clause 59: A method comprising:
receiving, at a computing device, location information associated with a catheter tip disposed within a heart of a subject;
receiving, at the computing device, imaging information, wherein at least a portion of the imaging information comprises first image data indicative of at least a portion of an endocardial surface of the heart, and wherein the at least a portion of the image information comprises second image data indicative of at least a portion of an epicardial surface of the heart;
determining, based on at least the first image data, a first distance indicative of a distance from the catheter tip to the at least the portion of the endocardial surface in a first direction;
determining, based on at least the second image data, a second distance indicative of a distance from the catheter tip to the at least the portion of the epicardial surface in the first direction;
determining a tissue thickness based at least on the first distance and the second distance; and
causing, via a display, output of thickness information indicative of the tissue thickness.

Example Clause 60: The method of Clause 59, wherein the output of the thickness information is indicated by a first color.

Example Clause 61: The method of Clause 60, wherein a first portion in the information indicative of the tissue thickness is bounded by a first point and a second point, wherein the first point comprises a point where a hypothetical line from the catheter tip extending in the first direction touches the endocardial surface, wherein the second point comprises a point where the hypothetical line touches the epicardial surface, wherein the first portion comprises the hypothetical line between the first point and the second point, and wherein the first portion comprises the first color.

Example Clause 62: The method of Clause 61, wherein the tissue thickness satisfies a thickness threshold and the first color is green.

Example Clause 63: The method of Clause 61, wherein the tissue thickness does not satisfy a thickness threshold and the first color is red.

Example Clause 64: The method of Clause 59, further comprising determining a confidence associated with the tissue thickness.

Example Clause 65: The method of Clause 64, further comprising:
comparing the confidence to a confidence threshold; and
causing an alert to be generated in response to determining that the confidence does not satisfy the confidence threshold.

Example Clause 66: The method of Clause 64, further comprising:
comparing the confidence to a confidence threshold; and
causing an indication to be generated in response to determining that the confidence satisfies the confidence threshold.

Example Clause 67: The method of Clause 59, wherein the location information comprises three-dimensional (3D) coordinate information.

Example Clause 68: A method comprising:
receiving, at a computing device, at least one coordinate set associated with a catheter tip;
receiving, at the computing device, at least one three-dimensional (3D) model of a heart;
determining, based on at least the at least one 3D model, an endocardial surface of the heart;
determining, based on at least the at least one 3D model, an epicardial surface of the heart;
causing a vector to be projected from the catheter tip, wherein the vector intersects the endocardial surface at a first point, and wherein the vector intersects the epicardial surface at a second point;
determining, using at least the first point and the second point, a tissue thickness; and causing, via a display, output of an indication of the tissue thickness.

Example Clause 69: The method of Clause 68, wherein the indication of the tissue thickness comprises a measurement.

Example Clause 70: The method of Clause 68, wherein the indication of the tissue thickness comprises a line along the vector from the first point to the second point.

Example Clause 71: The method of Clause 70, wherein the line comprises a color.

Example Clause 72: The method of Clause 71, wherein the tissue thickness satisfies a thickness threshold and the color is green.

Example Clause 73: The method of Clause 71, wherein the tissue thickness does not satisfy a thickness threshold and the color is red.

Example Clause 74: The method of Clause 68, further comprising causing, via the display, output of an indication of the catheter tip and the at least one 3D model.

Example Clause 75: The method of Clause 68, further comprising determining a confidence associated with the tissue thickness.

Example Clause 76: The method of Clause 75, further comprising:
comparing the confidence to a confidence threshold; and
causing an alert to be generated in response to determining that the confidence does not satisfy the confidence threshold.

Example Clause 77: The method of Clause 75, further comprising:
comparing the confidence to a confidence threshold; and
causing an indication to be generated in response to determining that the confidence satisfies the confidence threshold.

Example Clause 78: A method comprising:
receiving, at a computing device, at least one coordinate set associated with a catheter tip;
receiving, at the computing device, at least one three-dimensional (3D) model of a heart;
determining, based on the at least one 3D model, an endocardial surface of the heart;
determining, based on the at least one 3D model, an epicardial surface of the heart;
causing a first plane to be created in the at least one 3D model of the heart, wherein the first plane is associated with the endocardial surface;
causing a second plane to be created in the at least one 3D model of the heart, wherein the second plane is associated with the epicardial surface, and wherein the first plane is parallel to the second plane;
determining a first point on the first plane, wherein the first point is a closest point on the first plane to the catheter tip;
causing a vector to extend from the catheter tip through the first point and to a second point on the second plane;
determining a tissue thickness based at least on a distance along the vector from the first point to the second point; and
causing, via a display, output indicative of the tissue thickness.

Example Clause 79: The method of Clause 78, wherein the output indicative of the tissue thickness comprises a measurement.

Example Clause 80: The method of Clause 78, wherein the output indicative of the tissue thickness comprises a line along the vector from the first point to the second point.

Example Clause 81: The method of Clause 80, wherein the line comprises a color.

Example Clause 82: The method of Clause 81, wherein the tissue thickness satisfies a thickness threshold and the color is green.

Example Clause 83: The method of Clause 81, wherein the tissue thickness does not satisfy a thickness threshold and the color is red.

Example Clause 84: The method of Clause 78, further comprising determining a confidence associated with the tissue thickness.

Example Clause 85: The method of Clause 84, further comprising:
comparing the confidence to a confidence threshold; and
causing an alert to be generated in response to determining that the confidence does not satisfy the confidence threshold.

Example Clause 86: The method of Clause 84, further comprising:
comparing the confidence to a confidence threshold; and
causing an indication to be generated in response to determining that the confidence satisfies the confidence threshold.

Example Clause 87: The method of Clause 78, further comprising causing, via the display, output indicative of the endocardial surface, the epicardial surface, the catheter tip, the first point, and the second point.

The foregoing disclosure provides illustration and description but is not intended to be exhaustive or to limit the implementations to the precise form disclosed. Modifications may be made in light of the above disclosure or may be acquired from practice of the implementations. As used herein, the term "component" is intended to be broadly construed as hardware, firmware, or a combination of hardware and software. It will be apparent that systems and/or methods described herein may be implemented in different forms of hardware, firmware, and/or a combination of hardware and software. The actual specialized control hardware or software code used to implement these systems and/or methods is not limiting of the implementations. Thus, the operation and behavior of the systems and/or methods are described herein without reference to specific software code - it being understood that software and hardware can be used to implement the systems and/or methods based on the description herein. As used herein, satisfying a threshold may, depending on the context, refer to a value being greater than the threshold, greater than or equal to the threshold, less than the threshold, less than or equal to the threshold, equal to the threshold, and/or the like, depending on the context. Although particular combinations of features are recited in the claims and/or disclosed in the specification, these combinations are not intended to limit the disclosure of various implementations. In fact, many of these features may be combined in ways not specifically recited in the claims and/or disclosed in the specification

Although each dependent claim listed below may directly depend on only one claim, the disclosure of various implementations includes each dependent claim in combination with every other claim in the claim set. No element, act, or instruction used herein should be construed as critical or essential unless explicitly described as such. Also, as used herein, the articles "a" and "an" are intended to include one or more items and may be used interchangeably with "one or more." Further, as used herein, the article "the" is intended to include one or more items referenced in connection with the article "the" and may be used interchangeably with "the one or more." Furthermore, as used herein, the term "set" is intended to include one or more items (e.g., related items, unrelated items, a combination of related and unrelated items, and/or the like), and may be used interchangeably with "one or more." Where only one item is intended, the phrase "only one" or similar language is used. Also, as used herein, the terms "has," "have," "having," or the like are intended to be openended terms. Further, the phrase "based on" is intended to mean "based, at least in part, on" unless explicitly stated otherwise. Also, as used herein, the term "or" is intended to be inclusive when used in a series and may be used interchangeably with "and/or," unless explicitly stated otherwise (e.g., if used in combination with "either" or "only one of").

## Claims

1. : A system including one or more processors, configured to:
receive, at a computing device, location information associated with a catheter tip disposed within a heart of a subject;
receive, at the computing device, imaging information, where at least a portion of the imaging information includes first image data indicative of at least a portion of an endocardial surface of the heart, and where the at least a portion of the image information includes second image data indicative of at least a portion of an epicardial surface of the heart;
determine, based on at least the first image data, a first distance indicative of a distance from the catheter tip to the at least the portion of the endocardial surface in a first direction;
determine, based on at least the second image data, a second distance indicative of a distance from the catheter tip to the at least the portion of the epicardial surface in the first direction;
determine a tissue thickness based at least on the first distance and the second distance; and
cause, via a display, output of thickness information indicative of the tissue thickness.

2. : The system of claim 1, where the output of the thickness information is indicated by a first color.

3. : The system of claim 2, where a first portion in the information indicative of the tissue thickness is bounded by a first point and a second point, where the first point includes a point where a hypothetical line from the catheter tip extending in the first direction touches the endocardial surface, where the second point includes a point where the hypothetical line touches the epicardial surface, where the first portion includes the hypothetical line between the first point and the second point, and where the first portion includes the first color.

4. : The system of claim 2 or claim 3, where the tissue thickness satisfies a thickness threshold and the first color is green.

5. : The system of claim 2 or 3, where the tissue thickness does not satisfy a thickness threshold and the first color is red.

6. : The system of any one of claims 1 to 5, further including determining a confidence associated with the tissue thickness.

7. : The system of claim 6, further including : comparing the confidence to a confidence threshold; and causing an alert to be generated in response to determining that the confidence does not satisfy the confidence threshold.

8. : The system of claim 6, further including: comparing the confidence to a confidence threshold; and causing an indication to be generated in response to determining that the confidence satisfies the confidence threshold.

9. : The system of any one preceding claim, where the location information includes three-dimensional (3D) coordinate information.
